# EUROPEAN PATENT APPLICATION

(11) **EP 1 901 068 A1**
(43) Date of publication of application: **19.03.2008**
(21) Application number: 06120776.7
(22) Date of filing: 15.09.2006
(51) Int. Cl.: G01N 33/569

(54) **Methods of selecting cell clones**

(71) Applicant: Boehringer Ingelheim Pharma GmbH & Co. KG, 55216 Ingelheim am Rhein (DE)
(72) Inventor: Kaufmann, Hitto, 89075, Ulm (DE); Fieder, Jürgen, 89619, Unterstadion (DE)
(74) Representative: Hammann, Heinz

(57) **Abstract**

The invention describes novel methods for selecting cell clones which produce high amounts of protein of interest. In one method the amount of protein is measured before the cells are passaged for the first time. In another method a high throughput automated platform is used under sterile environment conditions with class A particle load of less than 100 particles per m3.

## Description

### BACKGROUND OF THE INVENTION

### TECHNICAL FIELD

The invention concerns the field of cell culture technology. It concerns a method of selecting cell clones as well as producer host cell lines selected thereby. The invention further concerns a method of producing proteins using the cells generated by the described screening method.
The invention additionally regards an automated platform for immediate-early high throughput screening, that means cell clone selection before the cells are passaged the first time, of mammalian cells producing proteins, especially therapeutic proteins, especially antibodies.

### BACKGROUND

The market for biopharmaceuticals for use in human therapy continues to grow at a high rate with 270 new biopharmaceuticals being evaluated in clinical studies and estimated sales of 30 billions in 2003 (Werner 2004). Currently, an increasing number of biopharmaceuticals is produced from mammalian cells due to their ability to correctly process and modify human proteins. Successful and high yield production of biopharmaceuticals from eukaryotic, especially mammalian cells is thus crucial and depends on the characteristics of the recombinant monoclonal cell line used in the process. In addition, the time to generate such a mammalian cell line producing a therapeutic protein is an essential part of the time needed to bring any biopharmaceutical to the clinic. Taken all these aspects together, there is an urgent need to develop methods to screen novel producer cell lines as fast as possible while maintaining the quality of the cell lines resulting from such a screen, particularly with regard to their productivity.

Generation of mammalian production cells generally requires a cloning step to ensure the population of cells grown in a bioreactor is genetically as homogeneous as possible. Limited dilution is a simple and well established method to generate monoclonal cell lines. However, when used for cloning suspension cells, its major caveat is the necessity to repeat the dilution step at least twice to reliably obtain populations that truly originate from a single parental cell. An attractive and reliable alternative is the use of fluorescence activated cell sorting (FACS) to generate monoclonal mammalian cell lines (WO2005019442).

Commonly used mammalian production cells constitutively secrete their product into the culture medium. The most common methods to detect and quantify the content of recombinant proteins in cell cultures are ELISA methods for detection of IgG type antibodies. While ELISAs offers very sensitive detection and quantification of proteins, such protocols are generally time consuming and include many steps. These properties make this assay format less attractive for automation and high-throughput screening. A number of alternative methods have been described to replace ELISAs as assay for determination of recombinant protein concentrations in mammalian cell cultures (Baker et al., 2002). However, many of them, such as optical biosensors or rapid chromatography can not yet be implemented for high-throughput screening of cell culture supernatants at early stages of cell line development.

Generally, characterization of newly generated monoclonal cell lines producing therapeutic proteins requires that samples are taken from the supernatant to be analyzed for metabolic parameters, product content and product quality. While some approaches have been described to increase amount and throughput of samples to be analyzed during cultivation of mammalian cells, these concept did not enable such measurement at the immediate-early stages of clone screening, that means before the cells are passaged the first time. (Lütkemyer et al., 2000).
The standard procedures used are not only time consuming but they also involve a high effort in cell culture maintainance and thus in cost.

There was, therefore, the need to accelerate this process of selecting cell clones which express high amounts of protein of interest.
Furthermore, there was the need to accelerate the process for the generation of high producer cell lines.

### SUMMARY OF THE INVENTION

Here we describe a novel method for selecting cell clones before passaging them for the first time, whereby said cell clones express high amounts of protein of interest. Furthermore, we describe a novel automated set-up for rapid high-throughput screening of cells such as Chinese hamster ovary (CHO) cells producing proteins such as therapeutic antibodies in serum-free and/or chemically defined media. The set-up consists of FACS-based single-cell cloning linked to a robotic station or automated platform performing an assay such as a homogenous time resolved fluorescence (HTRF^{®}) assay to detect the protein (antibody) content in monoclonal (CHO) cultures as they grow up from a single cell preferably in 96-well plates. As a key to efficient use of such an automated screening platform we further describe the use of autologous feeder cells to achieve high cloning efficiencies in chemically defined serum-free media. This concept represents the first automated platform for immediate-early clone-screening and will, therefore, serve as an essential step for increasing throughput in cell line development in the near future.

Homogeneous time-resolved fluorescent ("HTRF^{®}") assays have the advantage that they are homogeneous, sensitive, versatile, reproducible, safe, and robust. We have employed this assay format to replace a standard time-consuming ELISA format for detection of lgG type antibodies. We have designed a novel concept to enable the earliest possible screening of newly generated monoclonal production cell lines for their recombinant protein productivity. At the same time, the data demonstrate how this concept can expand the capacity of such a immediate early screen through automation. A single unit could potentially screen thousands of clones in 10-20 days.
In light of this observation the novel fast-track quality assessment for monoclonal cell line productivities described here will allow drastically reduced development times that are needed to establish reliable procedures for generation for new production cell lines.

The earliest possible screening step post single cell cloning is the analysis of primary monoclonal cell cultures, cultures of single cells giving rise to a cell line before they are passaged for the first time, herein also called immediate early screening. As these are the parental cultures of the cell cultures that will ultimately give rise to a master cell bank (MCB) as the primary stock for production of therapeutic protein, a crucial prerequisit of the present invention is the continued sterility during the whole screening procedure. This is a key challenge, which high throughput screens using automated platforms used for other purposes such as target screening do not meet or at least not to this extend. A specific laminar flow hood construction was implemented to guarantee sterility during the automated detection and selection steps, meaning less than x particles per m3 air.

The present invention is not obvious from the prior art.

The concept of selecting cell clones before passaging them, that means immediate-early screening, has not been described before, especially not in the context of biopharmaceutical producer host cell line selection. It is not obvious to apply such selection before passaging the cells, since the unpassaged cells are very sensitive,with regard to culture robustness. Furthermore, great care has to be taken when handling such cultures with regard to potential contaminations as no back-up culture exists at this stage. It was completely unexpected that such unpassaged cells would produce protein of interest with a pattern relevance to clone screening (see example 2). Surprisingly, the amount of protein produced under such conditions was sufficient to be detected as early as few days (for example 15 days) post single cell cloning. This is an essential fact enabling clone screening before the cells are passaged the first time. The containers used for single cell deposit, e.g. 96-well plates, do not display the same diffusion profile as a larger container generally used during culturing and passaging the cells before the measurement of the amount of the protein of interest. The protein expression profile of unpassaged cells surprisingly and unexpectedly resembled the profile of cells in batch culture, although the cell culture parameters are not comparable. This is of particular advantage as the most common process formats employed to produce e.g. therapeutic proteins batch formats or batch-derived formats. It is generally agreed, that the quality of any clone selection highly depends on how representative the culture format used during selection is for the final production format.

Especially surprising is the fact, that protein expression profiles resembling batch cultures can be achieved in the small containers, especially in multi-well containers such as 96-well even without shaking or rotating the multi-well containers or stirring the culture medium inside.

Concepts for automation of sampling and sample management during cultivation of mammalian cells have been described in the literature (Lütkemeyder et al., 2002). However, non of these concepts allowed the screening of primary monoclonal cell cultures for their productivity of a recombinant protein. Monoclonal cells generally need to be cultured in small volumes before the first passage (e.g. 200µl) as they need to condition their own culture medium to survive. Therefore a strict requirement for removal of samples from such cultures is the limitation to small amounts (0.2 - 5 µl preferably 0.5 - 2 µl). The handling of such small volumes to achieve the required accuracy for a high-quality selection process requires the use of a robotic pipetting platform.

HTRF^{®} assays have been known in the art. They are homogeneous, sensitive, versatile, reproducible, safe, and robust and have been gaining popularity in recent years. Most current applications of the HTRF^{®} assay format are within the field of drug screening (Mellor et al 1998). (www.htrf-assays.com).
However, none of the prior art documents concerning HTRF^{®} assays gives a hint towards application in screening host cell lines for production of proteins, e.g. recombinant proteins or in a method of selecting cell clones.

### DESCRIPTION OF THE FIGURES

FIGURE 1:
   A) Schematic of a standard method for selecting cell clones.
   B) chematic of integration of FACS and a robotic unit for immediate-early clone screening in cell line development:
      The earliest possible screen for productivity of novel monoclonal cell lines is conducted while single cells deposited by FACS grow up to cell populations in 96-wells. This concept requires the integration of an automated 96-well incubator into a sterile unit performing automated titer measurements in regular intervals.
FIGURE 2:
   Comparison of ELISA and HTRF^{®} based measurements of antibody concentration in 96-well and 384-well assay formats:
   CHO DG44 monoclonal cell lines producing an IgG type antibody were cultured in chemically defined serum-free media in 96-well plates. Supernatants were collected and the concentration of antibody in the culture media was determined by a sandwich-type anti lgG ELISA in a 96-well format and simultaneously by HTRF in an 96-well and an 384-well assay format. The two antibodies used in the ELISA and HTRF^{®} formats were from the same source.
FIGURE 3
   Schematic concept of an automated platform for HTRF^{®}-based titer measurements: 96-well plates containing single cells are transferred from a FACS unit to an automated incubator. The software schedules transfer of single plates from the incubator via an airlock into a sterile environment. Supernatants are removed and diluted by a pipetting unit while the cells are transferred back to the incubator. The pipetting unit then mixes sample and HTRF^{®} reagents in 384-well plates and transfers them to the storage hotel for incubation. After 2 hours the plates are moved to the reader for measurement at 665 nm and 620 nm. Sample tracking is ensured by barcoded plates and barcode readers.
FIGURE 4
   Titer curves obtained by automated HTRF^{®}-based immediate early screening of CHO cell clones
   Stable CHO cell pools expressing an IgG type 4 therapeutic antibody were single-cell deposited into 96-wells by FACS. Cells were transferred to the automated incubator and the automated titer measurement program was initiated 15 days post single cell sorting. Antibody titers were measured every three days for each well.

### DETAILED DESCRIPTION OF THE INVENTION

The general embodiments "comprising" or "comprised" encompass the more specific embodiment "consisting of. Furthermore, singular and plural forms are not used in a limiting way.
Terms used in the course of this present invention have the following meaning.

The term "immediate-early" means a point in time during the generation of a monoclonal cell line, where the monoclonal culture is still a primary culture and has not been passaged yet. That is, the single parental cell clone has been placed into a vial, where it has divided several times and has turned into a monoclonal cell population without having been split yet. The period of time which is termed as been "immediate-early" and where the cells have not been passaged yet can rank from 0-60 days, preferably from 1-60 days, more preferably from 1-30 days or from 5-60 days or from 5-30 days or from 5-25 days or from 10-25 days, and most preferably from 14-25 days.

The term "primary culture" means the initial culture step directly post single cell deposition e.g. by FACS or by limited dilution.

A "monoclonal cell line" means a cell line were all cells derive from a single parental cell. A monoclonal production cell line means a cell line producing a recombinant protein were all cells derive from a single parental cell.

"Automated" means that at least one step is performed without manual handling. The sequential operations are scheduled by a computer program.

"Automated platform" means a platform consisting of different instruments were the process that is performed on the platform is fully or semi-automated.

"Multi-well" means a cell culture device consisting of several equivalent culture vials, typically 6, 12, 24, 96 or 384 wells.

"Sterile" or "sterile environment" is defined by a class A particle load of less than 100 particles per m3. The sterile environment is preferentially generated by a laminar flow hood.

Incubator means a container for incubation of cells, preferably mammalian cells at a temperature of 37C +/- 5 °C and a CO₂ content of 3 - 12 %, preferably 5 - 10 %. The incubator is preferably an automated incubator enabling the sequential or scheduled presentation or transfer of cell culture vials to an automated platform.

"Fluorescence resonance energy transfer" ("FRET") means a process which uses two fluorophores, a donor and an acceptor. Excitation of the donor by an energy source (e.g. flash lamp or fluorometer laser) triggers an energy transfer towards the acceptor if they are within a given proximity to each other. The acceptor in turn emits light at its given wavelength.
Because of this energy transfer, molecular interactions between biomolecules can be assessed by coupling each partner with a fluorescent label and detecting the level of energy transfer. More importantly acceptor emissions, as a measure of energy transfer, can be detected without the need to separate bound from unbound complexes.
"Fluorescence resonance energy transfer" ("FRET") is a process by which a fluorophore donor in an excited state may transfer its excitation energy to a neighbouring chromophore acceptor non-radioactively through dipole-dipole interactions. In principle, if one has a donor molecule whose fluorescence emission spectrum overlaps the absorbance spectrum of a fluorescent acceptor molecule, they can exchange energy between one another through a non-radioactive dipole-dipole interaction. This energy transfer manifests itself by both quenching of donor fluorescence in the presence of acceptor and increased emission of acceptor fluorescence. Energy transfer efficiency varies most importantly as the inverse of the sixth power of the distance separating the donor and acceptor chromophores. The critical distance is the so- called Forster distance (usually between 10-1 00 Angstrom). The phenomenon can be detected by exciting the labeled specimen with light of a wavelength corresponding to the maximal absorption (excitation) of the donor and detecting light emitted at the wavelengths corresponding to the maximal emission of the acceptor, or by measuring the fluorescent lifetime of the donor in the presence and absence of the acceptor. The dependence of the energy transfer efficiency on the donor- acceptor separation provides the basis for the utility of this phenomenon in the study of cell component interactions. The conditions that need to exist for FRET to occur are: (1) the donor must be fluorescent and of sufficiently long lifetime; (2) the transfer does not involve the actual reabsorption of light by the acceptor; and (3) the distance between the donor and acceptor chromophores needs to be relatively close (usually within 10-50 Angstrom) (Herman, 1998, Fluorescence Microscopy, Bios scientific publishers, Springer, 2nd edition, page 12)

A further possibility to generate a signal is given with the so called "bioluminescence energy transfer" (BRET) system. This system is described in Arai et al., 2001, Anal, Biochem. 289 (I), 77-81. Said BRET system can also be used for the present invention and its sensitivity can be even higher than that of FRET. The example given in Arai et al. comprises Renilla luciferase, (Rluc) and enhanced yellow fluorescent protein (EYFP). Further, intramolecular energy transfer has been shown between Renilla luciferase (Rluc) and Aequorea "green fluorescent protein" (GFP) (Wang et al. 2002, Mol. Genet. Genomics 268(2), 160-8). In the presence of the luciferase substrate coelenterazine a GFP emission could be measured at the wave length of 508 nm, without UV excitation. Thus a "double emission" at 475 nm (luciferase) and 508 nm (GFP) could be measured. Furthermore, donor acceptor interactions in the systematically modified lanthanides such as Ru(II)-Os(II) have been described (Hurley & Tor, 2002, J. Am. Chem. SOC. 124(44), 1323-13241). Analyzes showed a Forster dipole-dipole energy transfer mechanism.

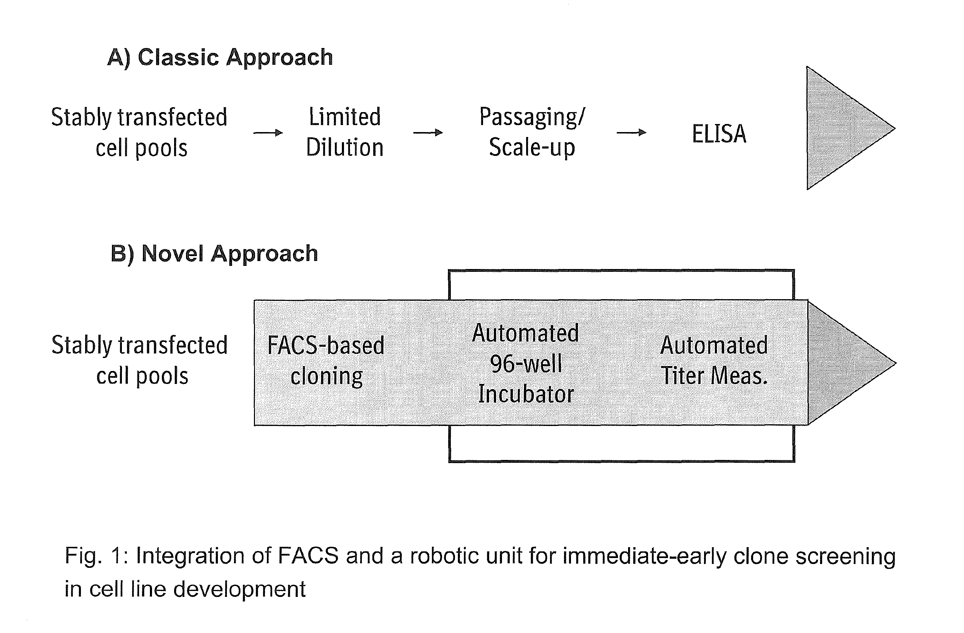

"FACS" means fluorescence activated cell sorting (see Herzenberg LA, Sweet RG, Herzenberg LA. Fluorescence-activated cell sorting. Sci Am 1976;234:108-117). The employment of "fluorescence activated cell sorting" ("FACS") allows a significant cut in process development times as only a single cloning step is required due to its accuracy. The concept described here, consists of a setup were clones are screened for their productivity at the earliest possible stage. Figure 1B describes such an immediate-early screen were the product titer of culture supernatants of cells growing in 96-wells subsequent to FACS-based single cell deposition is measured. Furthermore the titer measurements occur in a fully automated manner in a 384-well format to allow high-throughput primary screening for high-producer clones. Figure 1A in comparison shows a schematic of the standard method for selecting cell clones.

"HTRF^{®}" assays are "homogeneous time-resolved fluorescence assays" that generate a signal by FRET between donor and acceptor molecules.
HTRF^{®} (homogeneous time resolved fluorescence) is a technology based on TR-FRET, a combination of FRET chemistry and the use of fluorophores with long emission half-lives. While HTRF^{®} is based on TR-FRET chemistry it has many properties that separate it from other TR-FRET products. These include the use of a lanthanide with an extremely long half-life (Europium), conjugation of Eu3+ to cryptate, an entity which confers increased assay stability and the use of a ratiometric measurement that allows correction for quenching and sample interferences. Other HTRF^{®} technology features include homogeneous assay format, low background, simplified assay miniaturization, tolerance of additives such as DMSO & EDTA, few false positive/false negatives, cell-based functional assay.
In the HTRF^{®} assay, the donor is a Eu3+ caged in a polycyclic cryptate (Eu-cryptate), while the acceptor is a modified allophycocyanin protein. Laser excitation of the donor at 337 nm results in the transfer of energy to the acceptor at 620 nm when they are in close proximity (690 A ° ), leading to the emission of light at 665 nm over a prolonged period of milliseconds. A 50-Is time delay in recording emissions, and analyzing the ratio of the 665- and 620-nm emissions minimizes interfering fluorescence from the media and unpaired fluorophores.

In a specific embodiment the HTRF assay may serve to detect the content of IgG type antibodies in culture medium. In this case the Eu-cryptate is conjugated to anti human IgG antibody specifically binding to the Fc region and is presented upon binding of the antibody to the lgG product, while anti human lgG antibody specifically binding the kappa light chain is labelled as D2 acceptor to complete the complex.

The term "cell culture" means multiple cells cultivated in one container under conditions suitable for the growth of the cells.

"Suspension" culture means a suspension of cultured cells that have the potential to grow in liquid medium and do not attach to supportive surfaces of typical cell culture vessels. Some of these cells may have been adapted to gain such properties over a period of time.

The term "cloning" in the context of cell culture technology means a process whereby single cells are selected or isolated out of large cell populations. All daughter cells of such a single parental cell are identical / genetically identical.

The term "high throughput" means at least 250 measurements of protein concentration within 12 hours, preferably 500 measurements within 12 hours, more preferably 2000 measurements within 12 hours, most preferably 4000 measurements within 12 hours. This is calculated by the capacity of the multi-well plate used, e.g. 96-well plate multiplied by the number of plates fitting into the automated incubator relative to the performance speed of the automated platform measuring the samples. By using two incubators or larger incubators the throughput can be increased accordingly to 8000 measurements within a day or more. Using a time curve of measurements every three days, the throughput could be increased by using more incubator capacity to at least 24000 measurements within 3 days.

"Host cells" in the meaning of the present invention are cells such as hamster cells, preferably BHK21, BHK TK-, CHO, CHO-K1, CHO-DUKX, CHO-DUKX B1, and CHO-DG44 cells or the derivatives/progenies of any of such cell line. Particularly preferred are CHO-DG44, CHO-DUKX, CHO-K1 and BHK21, and even more preferred CHO-DG44 and CHO-DUKX cells. In a further embodiment of the present invention host cells also mean murine myeloma cells, preferably NS0 and Sp2/0 cells or the derivatives/progenies of any of such cell line.

Examples of murine and hamster cells which can be used in the meaning of this invention are also summarized in Table 1. However, derivatives/progenies of those cells, other mammalian cells, including but not limited to human, mice, rat, monkey, and rodent cell lines, or eukaryotic cells, including but not limited to yeast, insect, avian and plant cells, can also be used in the meaning of this invention, particularly for the production of biopharmaceutical proteins.

**TABLE 1: Hamster and murine production cell lines**

| CELL LINE | ORDER NUMBER |
|---|---|
| NS0 | ECACC No. 85110503 |
| Sp2/0-Ag14 | ATCC CRL-1581 |
| **BHK21** | ATCC CCL-10 |
| BHK TK⁻ | ECACC No. 85011423 |
| HaK | ATCC CCL-15 |
| 2254-62.2 (BHK-21 derivative) | ATCC CRL-8544 |
| CHO | ECACC No. 8505302 |
| CHO-K1 | ATCC CCL-61 |
| CHO-DUKX (= CHO duk⁻, CHO/dhfr⁻) | ATCC CRL-9096 |
| CHO-DUKX B1 | ATCC CRL-9010 |
| CHO-DG44 | Urlaub et al., Cell 33[2], 405-412, 1983 |
| CHO Pro-5 | ATCC CRL-1781 |
| V79 | ATCC CCC-93 |
| B14AF28-G3 | ATCC CCL-14 |
| CHL | ECACC No. 87111906 |

Host cells are most preferred, when being established, adapted, and completely cultivated under serum free conditions, and optionally in media which are free of any protein/peptide of animal origin. Commercially available media such as Ham's F12 (Sigma, Deisenhofen, Germany), RPMI-1640 (Sigma), Dulbecco's Modified Eagle's Medium (DMEM; Sigma), Minimal Essential Medium (MEM; Sigma), Iscove's Modified Dulbecco's Medium (IMDM; Sigma), CD-CHO (Invitrogen, Carlsbad, CA), CHO-S-Invtirogen), serum-free CHO Medium (Sigma), and protein-free CHO Medium (Sigma) are exemplary appropriate nutrient solutions. Any of the media may be supplemented as necessary with a variety of compounds examples of which are hormones and/or other growth factors (such as insulin, transferrin, epidermal growth factor, insulin like growth factor), salts (such as sodium chloride, calcium, magnesium, phosphate), buffers (such as HEPES), nucleosides (such as adenosine, thymidine), glutamine, glucose or other equivalent energy sources, antibiotics, trace elements. Any other necessary supplements may also be included at appropriate concentrations that would be known to those skilled in the art. In the present invention the use of serum-free medium is preferred, but media supplemented with a suitable amount of serum can also be used for the cultivation of host cells. For the growth and selection of genetically modified cells expressing the selectable gene a suitable selection agent is added to the culture medium.

The term "protein" is used interchangeably with amino acid residue sequences or polypeptide and refers to polymers of amino acids of any length. These terms also include proteins that are post-translationally modified through reactions that include, but are not limited to, glycosylation, acetylation, phosphorylation or protein processing. Modifications and changes, for example fusions to other proteins, amino acid sequence substitutions, deletions or insertions, can be made in the structure of a polypeptide while the molecule maintains its biological functional activity. For example certain amino acid sequence substitutions can be made in a polypeptide or its underlying nucleic acid coding sequence and a protein can be obtained with like properties.

The expression vector having a gene of interest encoding a protein of interest may also contain a selectable amplifiable marker gene.
The "selectable amplifiable marker gene" usually encodes an enzyme which is required for growth of eukaryotic cells under those conditions. For example, the selectable amplifiable marker gene may encode DHFR which gene is amplified when a host cell transfected therewith is grown in the presence of the selective agent, methotrexate (MTX). The non-limited exemplary selectable genes in Table 3 are also amplifiable marker genes, which can be used to carry out the present invention. For a review of the selectable amplifiable marker genes listed in Table 3, see Kaufman, Methods in Enzymology, 185:537-566 (1990), incorporated by reference. Accordingly, host cells genetically modified according to any method described herein are encompassed by this invention, wherein the selectable amplifiable marker gene encodes for a polypeptide having the function of dihydrofolate reductase (DHFR), glutamine synthetase, CAD, adenosine deaminase, adenylate deaminase, UMP synthetase, IMP 5'-dehydrogenase, xanthine guanine phosphoribosyl transferase, HGPRTase, thymidine kinase, thymidylate synthetase, P glycoprotein 170, ribonucleotide reductase, asparagine synthetase, arginosuccinate synthetase, ornithine decarboxylase, HMG CoA reductase, acetylglucosaminyl transferase, threonyl-tRNA synthetase or Na⁺K⁺-ATPase.

**TABLE 3: Selectable amplifiable marker genes**

| **Selectable Amplifiable Marker Gene** | **Accession Number** | **Selection Agent** |
|---|---|---|
| **DIHYDROFOLATE** | M19869 (hamster) | **METHOTREXATE (MTX)** |
| **REDUCTASE** | E00236 (mouse) | |
| Metallothionein | D10551 (hamster) | Cadmium |
| | M13003 (human) | |
| | M 11794 (rat) | |
| CAD (Carbamoyl-phosphate synthetase:Aspartate transcarbamylase: Dihydroorotase) | M23652 (hamster) | N-Phosphoacetyl-L-aspartate |
| | D78586 (human) | |
| Adenosine deaminase | K02567 (human) | Xyl-A- or adenosine, 2'deoxycoformycin |
| | M10319 (mouse) | |
| AMP (adenylate) deaminase | D12775 (human) | Adenine, azaserine, coformycin |
| | J02811 (rat) | |
| UMP synthase | J03626 (human) | 6-Azauridine, pyrazofuran |
| IMP 5'dehydrogenase | J04209 (hamster) | Mycophenolic acid |
| | J04208 (human) | |
| | M33934 (mouse) | |
| Xanthine-guanine phosphoribosyltransferase | X00221 (E. coli) | Mycophenolic acid with limiting xanthine |
| Mutant HGPRTase or mutant thymidine kinase | J00060 (hamster) | Hypoxanthine, aminopterin, and thymidine (HAT) |
| | M13542, K02581 (human) | |
| | J00423, M68489(mouse) | |
| | M63983 (rat) | |
| | M36160 (herpesvirus) | |
| Thymidylate synthetase | D00596 (human) | 5-Fluorodeoxyuridine |
| | M13019 (mouse) | |
| | L 12138 (rat) | |
| P-glycoprotein 170 (MDR1) | AF016535 (human) | Multiple drugs, e.g. adriamycin, vincristine, coichicine |
| | J03398 (mouse) | |
| Ribonucleotide reductase | M124223, K02927 (mouse) | Aphidicolin |
| Glutamine synthetase | AF150961 (hamster) | Methionine sulfoximine (MSX) |
| | U09114, M60803 (mouse) | |
| | M29579 (rat) | |
| Asparagine synthetase | M27838 (hamster) | β-Aspartyl hydroxamate, Albizziin, 5'Azacytidine |
| | M27396 (human) | |
| | U38940 (mouse) | |
| | U07202 (rat) | |
| Argininosuccinate synthetase | X01630 (human) | Canavanine |
| | M31690 (mouse) | |
| | M26198 (bovine) | |
| Ornithine decarboxylase | M34158 (human) | α-Difluoromethylornithine |
| | J03733 (mouse) | |
| | M 16982 (rat) | |
| HMG-CoA reductase | L00183, M12705 (hamster) | Compactin |
| | M11058 (human) | |
| N-Acetylglucosaminyl transferase | M55621 (human) | Tunicamycin |
| Threonyl-tRNA synthetase | M63180 (human) | Borrelidin |
| Na⁺K⁺-ATPase | J05096 (human) | Ouabain |
| | M 14511 (rat) | |

The present invention is suitable to generate host cells for the production of biopharmaceutical polypeptides/proteins. The invention is particularly suitable for the high-yield expression of a large number of different genes of interest by cells showing an enhanced cell productivity.
"Gene of interest", "selected sequence", or "product gene" have the same meaning herein and refer to a polynucleotide sequence of any length that encodes a product of interest or "protein of interest", also mentioned by the term "desired product". The selected sequence can be full length or a truncated gene, a fusion or tagged gene, and can be a cDNA, a genomic DNA, or a DNA fragment, preferably, a cDNA. It can be the native sequence, i.e. naturally occurring form(s), or can be mutated or otherwise modified as desired. These modifications include codon optimizations to optimize codon usage in the selected host cell, humanization or tagging. The selected sequence can encode a secreted, cytoplasmic, nuclear, membrane bound or cell surface polypeptide.
The "protein of interest" includes proteins, polypeptides, fragments thereof, peptides, all of which can be expressed in the selected host cell. Desired proteins can be for example antibodies, enzymes, cytokines, lymphokines, adhesion molecules, receptors and derivatives or fragments thereof, and any other polypeptides that can serve as agonists or antagonists and/or have therapeutic or diagnostic use. Examples for a desired protein/polypeptide are also given below.
The "product of interest" may also be an antisense RNA.

"Proteins of interest" or desired proteins are those mentioned above. Especially, desired proteins/polypeptides or proteins of interest are for example, but not limited to insulin, insulin-like growth factor, hGH, tPA, cytokines, such as interleukines (IL), e.g. IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (IFN) alpha, IFN beta, IFN gamma, IFN omega or IFN tau, tumor necrosisfactor (TNF), such as TNF alpha and TNF beta, TNF gamma, TRAIL; G-CSF, GM-CSF, M-CSF, MCP-1 and VEGF. Also included is the production of erythropoietin or any other hormone growth factors. The method according to the invention can also be advantageously used for production of antibodies or fragments thereof. Such fragments include e.g. Fab fragments (Fragment antigen-binding = Fab). Fab fragments consist of the variable regions of both chains which are held together by the adjacent constant region. These may be formed by protease digestion, e.g. with papain, from conventional antibodies, but similar Fab fragments may also be produced in the mean time by genetic engineering. Further antibody fragments include F(ab')2 fragments, which may be prepared by proteolytic cleaving with pepsin.

Using genetic engineering methods it is possible to produce shortened antibody fragments which consist only of the variable regions of the heavy (VH) and of the light chain (VL). These are referred to as Fv fragments (Fragment variable = fragment of the variable part). Since these Fv-fragments lack the covalent bonding of the two chains by the cysteines of the constant chains, the Fv fragments are often stabilised. It is advantageous to link the variable regions of the heavy and of the light chain by a short peptide fragment, e.g. of 10 to 30 amino acids, preferably 15 amino acids. In this way a single peptide strand is obtained consisting of VH and VL, linked by a peptide linker. An antibody protein of this kind is known as a single-chain-Fv (scFv). Examples of scFv-antibody proteins of this kind known from the prior art are described in Huston et al. (1988, PNAS 16: 5879-5883).

In recent years, various strategies have been developed for preparing scFv as a multimeric derivative. This is intended to lead, in particular, to recombinant antibodies with improved pharmacokinetic and biodistribution properties as well as with increased binding avidity. In order to achieve multimerisation of the scFv, scFv were prepared as fusion proteins with multimerisation domains. The multimerisation domains may be, e.g. the CH3 region of an IgG or *coiled coil* structure (helix structures) such as *Leucin-zipper* domains. However, there are also strategies in which the interaction between the VH/VL regions of the scFv are used for the multimerisation (e.g. dia-, tri- and pentabodies). By diabody the skilled person means a bivalent homodimeric scFv derivative. The shortening of the *Linker* in an scFv molecule to 5- 10 amino acids leads to the formation of homodimers in which an inter-chain VH/VL-superimposition takes place. Diabodies may additionally be stabilised by the incorporation of disulphide bridges. Examples of diabody-antibody proteins from the prior art can be found in Perisic et al. (1994, Structure 2: 1217-1226).

By minibody the skilled person means a bivalent, homodimeric scFv derivative. It consists of a fusion protein which contains the CH3 region of an immunoglobulin, preferably IgG, most preferably IgG1 as the dimerisation region which is connected to the scFv via a *Hinge region* (e.g. also from lgG1) and a *Linker* region. Examples of minibody-antibody proteins from the prior art can be found in Hu et al. (1996, Cancer Res. 56: 3055-61).

By triabody the skilled person means a: trivalent homotrimeric scFv derivative (Kortt et al. 1997 Protein Engineering 10: 423-433). ScFv derivatives wherein VHVL are fused directly without a linker sequence lead to the formation of trimers.

The skilled person will also be familiar with so-called miniantibodies which have a bi-, tri- or tetravalent structure and are derived from scFv. The multimerisation is carried out by di-, tri- or tetrameric coiled coil structures (Pack et al., 1993 Biotechnology 11:, 1271-1277; Lovejoy et al. 1993 Science 259: 1288-1293; Pack et al., 1995 J. Mol. Biol. 246: 28-34).

The invention regards a method of selecting cell clones characterized by the following steps
a) Depositing single cells expressing a protein of interest in individual containers in a culture medium,
b) Culturing the cells for at least one day,
c) Removing an aliquot of the culture from each container before the cells are passaged the first time,
d) Measuring the amount of the protein of interest in each aliquot,
e) Selecting clones according to the amount of protein measured in the respective aliquot.

A preferred embodiment is an inventive method wherein the throughput is at least 250 measurements (of protein concentration) within 12 hours, preferably 500 measurements within 12 hours, more preferably 2000 measurements within 12 hours, most preferably at least 4000 measurements or aliquots in 12 hours.

Another preferred embodiment of the invention is an inventive method wherein step c) is performed in a sterile environment class A particle load of less than 100 particles per m3.

A specific embodiment of the invention is an inventive method wherein at least one step is performed in multi-well plates as well as a method wherein at least step d) is performed in multi-well plates as well as a method wherein the multi-well plates are 96-well plates or 384-well plates, preferably 384-well plates.

Another preferred embodiment consists of a method wherein step a) is performed in 96-well plates and step d) is performed in 384-well plates.

A further preferred embodiment of the invention is an inventive method wherein the clones /clonal cultures are monitored over a period of time that is sufficient to obtain batch-like titer curves, preferably over a period of 5-15 days with samples taken every 2-3 days.

The invention furthermore concerns a method of selecting cell clones characterized by the following steps:
a. depositing single cells expressing a protein of interest in multi-well containers in a cell culture medium,
b. passaging the derived cell cultures up to 10 times,
c. transferring said multi-well containers to an automated incubator,
d. sequentially transferring said multi-well containers from the incubator via an airlock into a sterile environment having class A particle load of less than 100 particles per m3,
e. removing an aliquot of the culture from each container,
f. diluting the samples by a pipetting unit while the cells are transferred back to the incubator,
g. Mixing the diluted samples and the assay reagents into another multi-well container,
h. transferring the multi-well containers of step g) to the storage hotel for incubation,
i. Moving the multi-well plates of step h) to a reader,
j. measuring the amount of the protein of interest in each container,
whereby the throughput is at least 250 measurements within 12 hours, preferably 500 measurements within 12 hours, more preferably 2000 measurements within 12 hours, most preferably at least 4000 measurements or aliquots in 12 hours.

A preferred embodiment of the inventive method is a method wherein sample tracking is ensured by barcoded plates and barcode readers.

Another preferred embodiment is a method wherein the number of passages in step b) is 0 and step e) is performed before the cells are passaged the first time.

A further preferred embodiment is a method wherein the multi-well plates are 96-well plates or 384-well plates, preferably 384-well plates as well as a method wherein steps a) to e) are performed in 96-well plates and steps g) to j) are performed in 384-well plates.

A further specific embodiment is an inventive method wherein multi-well containers for culturing the monoclonal cells are removed from the incubator for a maximum time period of 5 minutes, and were in step e) the lid of the multi-well container is removed for no longer than 1 minute, preferably 30 seconds.

Another preferred embodiment is any of the inventive method wherein the cells of step a) have been transfected with an expression vector containing a gene of interest in order to express a protein of interest.

A specific embodiment is any of the inventive methods wherein the single cells have been generated by using fluorescence activated cell sorting (FACS) or by limited dilution.

Another specific embodiment is any of the methods wherein the culturing time in step b) of the first method and the time between one passage and another in step b) of the second method is between 1-60 days or 1-30 days or 5-60 days or 5-30 days or 10-60 days or 10-30 days or 5-30 days or 5-25 days or preferably 14-25 days.

A preferred embodiment is any of the inventive methods wherein the aliquot in step c) of the first method and the aliquot of step e) of the second method is from the cell culture supernatant.

Another preferred embodiment is any of the inventive methods wherein the measurement step is performed by an enzyme linked immuno-sorbent assay (ELISA) or preferably by an homogeneous time-resolved fluorescence assay (HTRF), preferably by HTRF and especially preferred is a method wherein the HTRF assay comprises detection antibodies directed to
a. the Fc part of lgG type antibodies and to
b. a light chain of lgG type antibodies.

A specifically preferred embodiment is any of the inventive methods wherein the detection antibodies are anti h lgG (Fc) conjugated to Europium cryptate donor and anti h kappa light chain conjugated to a D2 acceptor.

Another preferred embodiment is any of the inventive methods wherein the culture medium is serum-free and /or animal component-free and / or protein free and /or chemically defined.

Another especially preferred embodiment is any of the inventive methods wherein the cells are grown in suspension culture.

A further specific preferred embodiment is any of the inventive methods wherein the selected clones represent the top 30%, preferably the top 20% and most preferably the top 10% of cells measured to express high amounts of the protein of interest.

In another preferred embodiment of any of the inventive methods the method is performed without shaking or rotating the multi-well containers or stirring the culture medium inside.

Another preferred embodiment is any of the inventive methods wherein the method is further characterized by the use of autologous feeder cells. Preferably this is a method wherein the feeder cells used are hamster cells when the deposited cells are CHO- or BHK- cells and wherein maus-myeloma cells are used as feeder cells when the deposited cells are NSO cells. More preferably, this is a method wherein the deposited cells are grown in the presence of 100 to 200.000 *feeder*-cells per mL medium.

Another preferred embodiment is any of the inventive methods wherein the protein of interest is a therapeutic protein, preferably wherein the protein is an antibody, especially a therapeutic antibody.

Another specific embodiment is any of the inventive methods wherein the deposited cell is a hamster cell, e.g. CHO or BHK cell or wherein the deposited cell is a mouse myeloma cell, e.g. NSO cell.

The invention further concerns a method of increasing throughput in cell line development by using any of the previous methods of selecting cell clones.

The invention furthermore concerns a method of producing a protein in a eukaryotic cell, e.g. a mammalian cell, under serum-free culturing conditions characterized by the following steps:
a. Generating a eukaryotic cell which contains a gene of interest encoding a protein of interest,
b. Cultivating the cell under serum-free conditions, which allow the proliferation of the cell,
c. Deposition of single cells in a multi-well container, such as a 96-well plate ,
d. Cultivation of said single cells optionally in the presence of autologous feeder cells,
e. Screening the clonal cells according to any one of the inventive methods previously described,
f. Cultivating the top 30%, preferably the top 20% and most preferably the top 10% of selected cells measured to express high amounts of the protein of interest,
g. Harvesting the protein of interest e.g. by separating the cells from the supernatant and
h. Purifying the protein of interest.

A preferred embodiment is a method wherein the protein of interest is a recombinant protein, preferably a therapeutic protein, more preferably an antibody.

The invention additionally concerns a protein product produced by any one of the methods described.

The invention furthermore concerns a method of selecting a producer host cell line by using any one of the methods described.

The invention further concerns a producer host cell line selected by any of the methods described.

A specific embodiment is a producer host cell line wherein the host cell is a eukaryotic cell, especially a mammalian cell, preferably wherein the host cell is a hamster or a mouse-myeloma cell, especially a CHO- or BHK-cell or a NSO cell.

The invention furthermore concerns the use of a producer host cell line as described for biopharmaceutical protein manufacturing.

Additionally, the invention concerns a laminar flow hood suitable to establish a sterile environment supplying class A particle load of less than 100 particles / m3 and which is suitable for an automated platform performing any of the inventive methods as described.

The invention furthermore concerns a method of immediate-early high throughput screening of cells characterized by the following steps:
a) Performing single-cell cloning of transfected cells genetically modified to express a protein of interest and
b) Performing a protein detection assay suitable to detect said protein of interest in a primary cell culture of monoclonal cells growing in a multi-well plate format using an automated platform while
c) maintaining a sterile environment of the primary cell culture culture.

In a specific embodiment the invention further concerns a method of immediate-early high throughput screening of cells using an automated platform wherein the following steps are performed:
a. 96-well plates containing single cells are transferred from a FACS unit to an automated incubator,
b. The software sequentially schedules transfer of single plates from the incubator via an airlock into a sterile environment,
c. Supernatants are removed and diluted by a pipetting unit while the cells are transferred back to the incubator,
d. The pipetting unit then mixes sample and assay reagents in multi-well plates and transfers them to the storage hotel for incubation,
e. After 2 hours the plates are moved to the reader for measurement at the expected wave length.
f. Sample tracking is ensured by barcoded plates and barcode readers.

In a preferred embodiment of any of the inventive methods the amount of data obtained using an automated set up would enable the generation of typical titer profiles of each specific cell type under the above described culture conditions. These profiles could than be used to reduce the number of measurements needed for clone selection as they could enable extrapolations. This again would increase the possible maximum throughput of any such set up.
In case it is desired to limit the number of samples taken and/or the time frame for the selection procedure, the described setup would enable the generation of typical titer profiles that could be used to estimated the titer potential of such clones (mathematical modelling approach). Titer potential means the final protein concentration that the culture would reach before the first passaging.

The practice of the present invention will employ, unless otherwise indicated, conventional techniques of cell biology, molecular biology, cell culture, immunology and the like which are in the skill of one in the art. These techniques are fully disclosed in the current literature. See e.g. Sambrook et al., Molecular Cloning: A Laboratory Manual, 2nd Ed., Cold Spring Harbor Laboratory Press, Cold Spring Harbor, N.Y. (1989); Ausubel et al., Current Protocols in Molecular Biology (1987, updated); Brown ed., Essential Molecular Biology, IRL Press (1991); Goeddel ed., Gene Expression Technology, Academic Press (1991); Bothwell et al. eds., Methods for Cloning and Analysis of Eukaryotic Genes, Bartlett Publ. (1990); Wu et al., eds., Recombinant DNA Methodology, Academic Press (1989); Kriegler, Gene Transfer and Expression, Stockton Press (1990); McPherson et al., PCR: A Practical Approach, IRL Press at Oxford University Press (1991); Gait ed., Oligonucleotide Synthesis (1984); Miller & Calos eds., Gene Transfer Vectors for Mammalian Cells (1987); Butler ed., Mammalian Cell Biotechnology (1991); Pollard et al., eds., Animal Cell Culture, Humana Press (1990); Freshney et al., eds., Culture of Animal Cells, Alan R. Liss (1987); Studzinski, ed., Cell Growth and Apoptosis, A Practical Approach, IRL Press at Oxford University Presss (1995); Melamed et al., eds., Flow Cytometry and Sorting, Wiley-Liss (1990); Current Protocols in Cytometry, John Wiley & Sons, Inc. (updated); Wirth & Hauser, Genetic Engineering of Animals Cells, in: Biotechnology Vol. 2, Pühler ed., VCH, Weinheim 663-744; the series Methods of Enzymology (Academic Press, Inc.), and Harlow et al., eds., Antibodies: A Laboratory Manual (1987).

The invention generally described above will be more readily understood by reference to the following examples, which are hereby included merely for the purpose of illustration of certain embodiments of the present invention and are not intended to limit the invention in any way.

### EXAMPLES

### MATERIALS AND METHODS

### Cell Culture

All cell lines used at production and development scale were maintained in serial seedstock cultures in surface-aerated T-flasks (Nunc, Denmark) in incubators (Thermo, Germany) or spinner flasks sparged with a mixture of air and 5% CO₂ (Wheaton, USA) in specially designed incubator rooms at a temperature of 37°C.

Seedstock cultures were subcultivated every 2-3 days with seeding densities of 2E5- 3E5 cells/mL. The cell concentration was determined in all cultures by using a hemocytometer. Viability was assessed by the trypan blue exclusion method. The cultures originated from master, working or safety cell banks and were thoroughly tested for at least sterility, mycoplasma and the presence of adventitious viruses. All operations took place in air-filtered laboratories and under strict procedures complying to 'current Good Manufacturing Practices (cGMP)'. All CHO production cells were cultured in media and their composition proprietary to Boehringer Ingelheim.

Cell lines producing recombinant proteins (Protein of interest) were generated by stably transfecting plasmids containing DNA encoding the protein into CHO cells. Stable cell pools (polyclonal cell populations) were generated by applying a selection procedure suich as the one described in Sautter and Enenkel: Selection of high-producing CHO cells using NPT selection marker with reduced enzyme activity.Biotechnol Bioeng. 2005 Mar 5;89(5):530-8..

### Single Cell Sorting

A FACS Vantage (Coulter EPICS ALTRA HyPerSort System)) flow cytometer equipped with pulse processing, sort enhancement module, and automatic cell deposition unit was used for analysis and cell sorting. A Argon Laser (Coherent), tuned to 488 nm was used. Laser Output power was 220 mW. Viable cells were sorted by setting a gate including all single cells according toa dot plot of forward scatter (FSC) vs. side scatter (SSC). Sorted cells were deposited into 96-well microtiter plates containing 200 µl growth medium at two cells per well with the automatic cell deposition unit. For sterile sorting the tubing of the cell sorter was cleaned and sterilized by running as sheath fluid for 1 h each of the following solutions: 70% ethanol, sterile H₂O

### HTRF assay

"HTRF" assays are "homogeneous time-resolved fluorescence assays" that generate a signal by FRET between donor and acceptor molecules.
The donor is a Eu3+ caged in a polycyclic cryptate (Eu-cryptate), while the acceptor is a modified allophycocyanin protein. Laser excitation of the donor at 337 nm results in the transfer of energy to the acceptor at 620 nm when they are in close proximity (690 A ° ), leading to the emission of light at 665 nm over a prolonged period of milliseconds. A 50-ls time delay in recording emissions, and analyzing the ratio of the 665- and 620-nm emissions minimizes interfering fluorescence from the media and unpaired fluorophores. To detect the content of lgG type antibodies in culture medium, the Eu-cryptate was conjugated to anti human IgG antibody specifically binding to the Fc region and is presented upon binding of the antibody to the lgG product, while anti human IgG antibody specifically binding the kappa light chain was labelled as D2 acceptor to complete the complex.

### Anti h IgG (Fc) conjugation to Europium cryptate donor

The antibody was first dyalised in phosphate buffer 50mM pH8 and concentrated to 1mg/mL using Biomax tips (cut off 30 000 M.W) from Millipore. The antibody was then reacted with N-Hydroxy-succinimide activated cryptate for 30 minutes at room temperature in a molar ratio of 15 cryptate / antibody. The antibody cryptate conjugate was finally purified from the unreacted fluorophore on a G25 superfine gel.

### Anti h Kappa light chain conjugation to D2 acceptor

The antibody was first dyalised in phosphate buffer 50mM pH8.5 and concentrated to 1mg/mL using Biomax tips (cut off 30 000 M.W) from Millipore. The antibody was then reacted with N-Hydroxy-succinimide activated D2 for 1 hour at room temperature in a molar ratio of 5 D2 / antibody. The antibody D2 conjugate was finally purified from the unreacted fluorophore on a G25 superfine gel.

### EXAMPLE 1:

A robotic platform performing HRTF-based measurements of IgG antibodies in culture supernatants of CHO cells in a sterile environment.

Figure 1B shows the schematic of the immediate-early screen set up used. The product titer of culture supernatants of cells growing in 96-wells subsequent to FACS-based single cell deposition were measured. Furthermore the titer measurements occured in a fully automated manner in a 384-well format to allow high-throughput primary screening for high-producer clones.

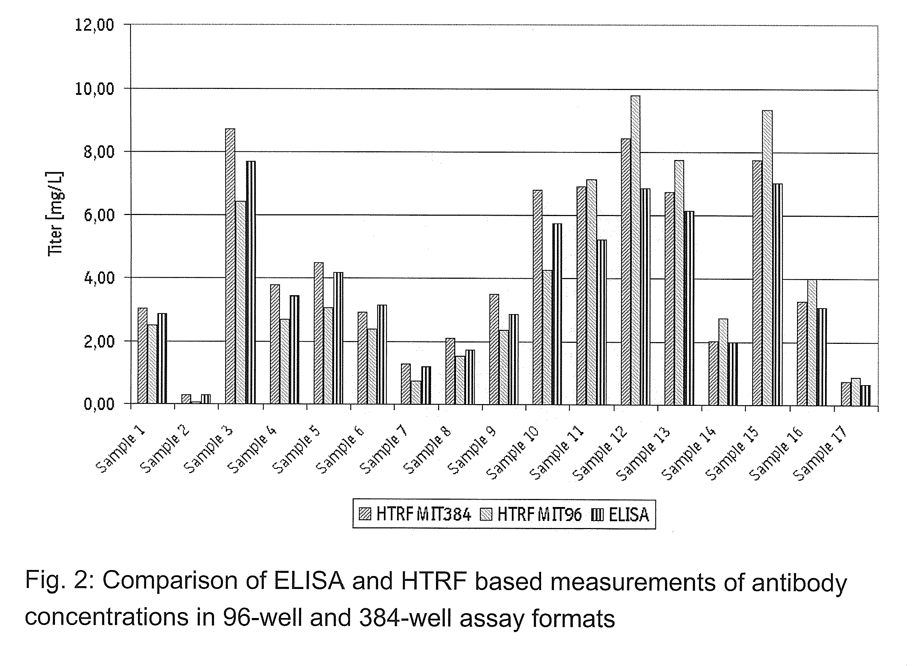

To evaluate the feasibility of using the described HTRF assay instead of the classic ELISA several IgG producing CHO cell populations were analyzed for antibody production with both assays side by side (Figure 2). In addition it was assessed how a shift from the current 96-well format to a 384-well format would affect the assay performance. Figure 2 shows a good correlation between the three assay formats for all cell populations over a wide range of absolute antibody concentrations from 0.025 to 10mg/l. Overall, any productivity-based ranking of the CHO cell populations based on the 384-well HTRF format gave the same result as employing the original ELISA format.

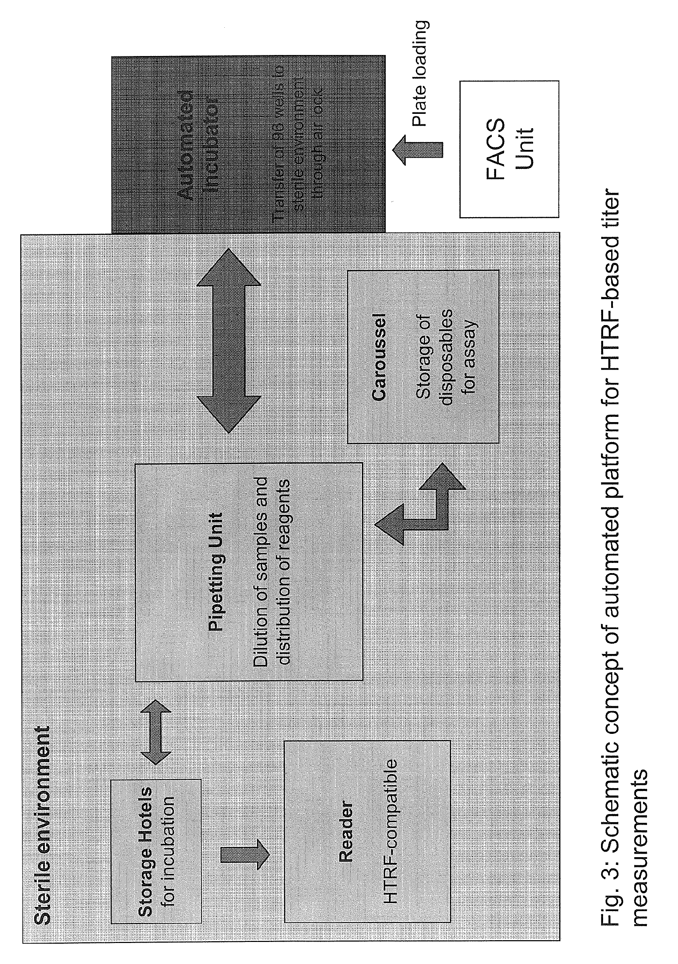

The 384-well HTRF format was automated and linked to a source incubator holding 42 96-well plates containing cell clones. A layout of the immediate-early clone screening platform is depicted in Figure 3 The platform consists of a Freedom EVO 200 basic module (Tecan, Switzerland), a pipetting unit consisting of Te-MO-96 3/5, Te-MO WRC and Te-MO Refill stations (Tecan Switzerland, an Ultra Evolution Reader (Tecan), a LPR240 Karussell (Liconics), a Cytomat 2C Incubator (Thermo) and a computing unit (Dell). The incubator sequentially presented all plates through an air lock to the central pipetting unit, were a sample of the each culture supernatant was taken. After an initial dilution step all further reactions took place in 384-wells. Samples were incubated with donor and acceptor solutions in four serial dilutions. Plates were incubated for 2 hours prior to measurement. The platform was optimized for the maximum throughput using the FACTS software (Tecan, Switzerland). This scheduling allowed HTRF-based antibody quantification from 42 cell culture source plates in approximately 12 hours. This would translate to the ability to screen more than 4000 monoclonal cell lines for antibody secretion in a single run. The current throughput would also allow another incubator unit to be screened within the same day. Assuming titer measurements every third day, the described automated screening platform could be further extended to screen 24000 supernatants of monoclonal cell lines simultaneously.

### EXAMPLE 2

### Automated immediate early screening of monoclonal CHO cells producing an IgG-4 type antibody

Genes encoding an lgG4 type antibody were transfected into CHO DG44 cells growing in chemically defined serum-free media and stable cell pools were generated by selection with neomycin. Cells were subjected to FACS-based single cell cloning including the use of autologous feeder cells as described above. After a period of time of 15 days post single cell cloning, 42 plates were transferred into an automated incubator and the immediate early clone screening program was initiated. Supernatants of all clonal cultures were taken every 3 days and the antibody concentration was measured by the described HTRF assay.

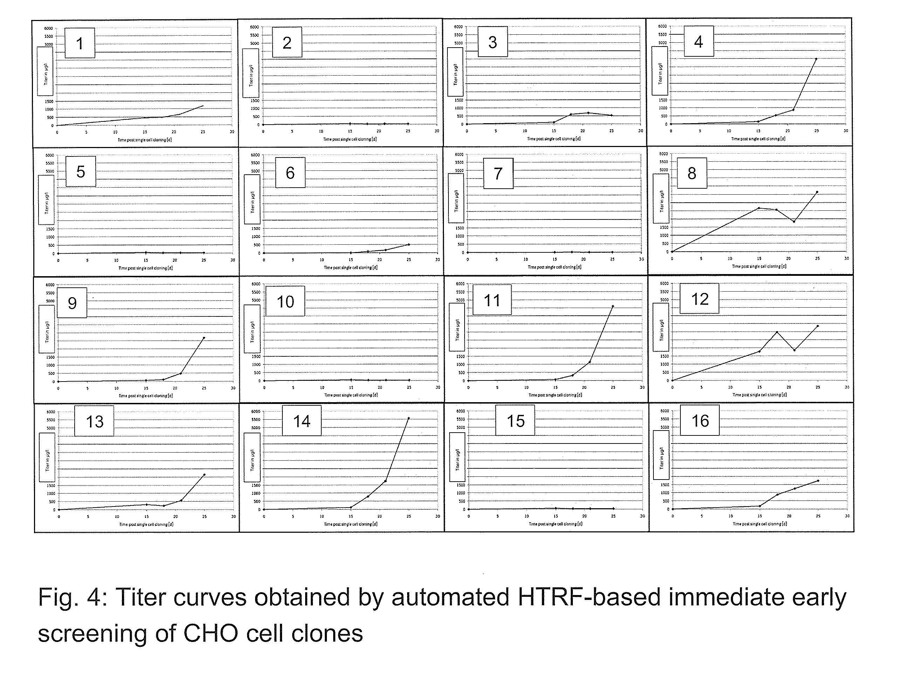

Figure 4 shows the results for 16 representative clonal CHO cultures (panel 1-16 as indicated) as they grow up from single cells in 96-wells. For most cultures, titer curves indicate that they enter exponential growth phase at around day 15 post single cell deposition (such as clones depicted in panel 4, 11 and 14). However, some cultures demonstrate faster growth kinetics as the antibody concentration has already reached a plateau level between day 15 and day 25, (such as clones depicted in panel 8 and 12)
Some cultures have just entered early exponential growth phase at the last point of measurement (such as clones depicted in panel 9 and 13).
In case it is desired to limit the number of samples taken and/or the time frame for the selection procedure, the described setup would enable the generation of typical titer profiles that could be used to estimated the titer potential of such clones (mathematical modelling approach).Titer potential means the final protein concentration that the culture would reach before the first passaging.

These data demonstrate how this immediate early screening concept can distinguish between high and low producer clones rapidly and enables to include many thousands of clones in this primary screen.

## Claims

1. A method of selecting cell clones **characterized by** the following steps
a. Depositing single cells expressing a protein of interest in individual containers in a culture medium,
b. Culturing the cells for at least one day,
c. Removing an aliquot of the culture from each container before the cells are passaged the first time,
d. Measuring the amount of the protein of interest in each aliquot,
e. Selecting clones according to the amount of protein measured in the respective aliquot.

2. A method according to claim 1 wherein the throughput is at least 250 measurements within 12 hours, preferably 500 measurements within 12 hours, more preferably 2000 measurements within 12 hours, most preferably at least 4000 measurements in 12 hours.

3. A method according to claims 1 or 2 wherein step c) is performed in a sterile environment with class A particle load of less than 100 particles per m3.

4. A method according to claims 1-3 wherein at least one step is performed in multi-well plates.

5. A method according to claims 4 wherein at least step d) is performed in multi-well plates.

6. A method according to claims 4 or 5 wherein the multi-well plates are 96-well plates or 384-well plates, preferably 384-well plates.

7. A method according to claims 4-6 wherein step a) is performed in 96-well plates and step d) is performed in 384-well plates.

8. A method of selecting cell clones **characterized by** the following steps:
a. depositing single cells expressing a protein of interest in multi-well containers in a cell culture medium,
b. passaging the derived cell cultures up to 10 times
c. transferring said multi-well containers to an incubator,
d. sequentially transferring said multi-well containers from the incubator via an airlock into a sterile environment having class A particle load of less than 100 particles per m3,
e. removing an aliquot of the culture from each container,
f. diluting the samples by a pipetting unit while the cells are transferred back to the incubator,
g. Mixing the diluted samples and the assay reagents into another multi-well container,
h. transferring the multi-well containers of step g) to the storage hotel for incubation,
i. Moving the multi-well plates of step h) to a reader,
j. measuring the amount of the protein of interest in each container,
k. whereby the throughput is at least 250 measurements within 12 hours, preferably 500 measurements within 12 hours, more preferably 2000 measurements within 12 hours, most preferably at least 4000 measurements in 12 hours.

9. A method according to claim 8 wherein sample tracking is ensured by barcoded plates and barcode readers.

10. A method according to claim 8 or 9 wherein the number of passages in step b) is 0 and step e) is performed before the cells are passaged the first time.

11. A method according to claims 8 to 10 wherein the multi-well plates are 96-well plates or 384-well plates, preferably 384-well plates.

12. A method according to claims 8 to 11 wherein steps a) to e) are performed in 96-well plates and steps g) to j) are performed in 384-well plates.

13. A method according to claims 1 to 12 wherein the cells of step a) have been transfected with an expression vector containing a gene of interest in order to express a protein of interest.

14. A method according to claim 1 to 13 wherein the single cells have been generated by using fluorescence activated cell sorting (FACS) or by limited dilution.

15. A method according to claims 1 to 14 wherein the culturing time in step b) of claim 1 and the time between one passage and another in step b) of claim 9 is between 1-60 days or 1-30 days or 5-60 days or 5-30 days or 10-60 days or 10-30 days or 5-30 days or 5-25 days or preferably 14-25 days.

16. A method according to claim 1 to 15 wherein the aliquot in step c) of claim 1 and the aliquot of step e) of claim 9 is from the cell culture supernatant.

17. A method according to claim 1 to 16 wherein the measurement step is performed by an enzyme linked immuno-sorbent assay (ELISA) or preferably an homogeneous time-resolved fluorescence assay (HTRF), preferably by HTRF.

18. A method according to claim 17 wherein the HTRF assay comprises detection antibodies directed to
a. the Fc part of lgG type antibodies and to
b. a light chain of lgG type antibodies

19. A method according to claim 18 wherein the detection antibodies are anti h lgG (Fc) conjugated to Europium cryptate donor and anti h kappa light chain conjugated to a D2 acceptor.

20. A method according to claim 1-19 wherein the culture medium is serum-free and /or animal component-free and / or protein free and /or chemically defined.

21. A method according to claim 1 to 20 wherein the cell is grown in suspension culture.

22. A method according to claim 1 to 21 wherein the selected clones represent the top 30%, preferably the top 20% and most preferably the top 10% of cells measured to express high amounts of the protein of interest.

23. A method according to claims 1-22 wherein the method is further **characterized by** the use of autologous feeder cells.

24. A method according to claim 23 wherein the feeder cells used are hamster cells when the deposited cells are CH- or BHK- cells and wherein maus-myeloma cells are used as feeder cells when the deposited cells are NSO cells.

25. A method according to claim 23 or 24 wherein the deposited cells are grown in the presence of 100 to 200.000 *feeder*-cells per mL medium.

26. A method of claims 1-25 wherein the protein of interest is a therapeutic protein.

27. A method according to claims 1-26 wherein the protein is an antibody, especially a therapeutic antibody.

28. A method according to claim 1-27 wherein the deposited cell is a hamster cell, e.g. CHO or BHK cell.

29. A method according to claim 1-27 wherein the deposited cell is a mouse myeloma cell, e.g. NSO cell.

30. Method of increasing throughput in cell line development by using a method according to claims 1-29.

31. A method of producing a protein in a eukaryotic cell, e.g. a mammalian cell, under serum-free culturing conditions **characterized by** the following steps:
a. Generating a eukaryotic cell which contains a gene of interest encoding a protein of interest,
b. Cultivating the cell under serum-free conditions, which allow the proliferation of the cell,
c. Deposition of single cells in a multi-well container, such as a 96-well plate ,
d. Cultivation of said single cells optionally in the presence of autologous feeder cells,
e. Screening the clonal cells according to any one of the methods of claims 1-30,
f. Cultivating the top 30%, preferably the top 20% and most preferably the top 10% of selected cells measured to express high amounts of the protein of interest,
g. Harvesting the protein of interest e.g. by separating the cells from the supernatant and
h. Purifying the protein of interest.

32. A method according to claim 31 wherein the protein of interest is a recombinant protein.

33. A method according to claim 31 or 32 wherein the protein of interest is a therapeutic protein.

34. A method according to claim 31 to 33 wherein the protein of interest is an antibody.

35. A protein product produced by any one of the methods described in claims 31-34.

36. A method of selecting a producer host cell line by using any one of the methods of claims 1-29.

37. A producer host cell line selected by the method of claim 36.

38. A producer host cell line according to claim 37 wherein the host cell is a eukaryotic cell, especially a mammalian cell.

39. A producer host cell line according to claim 38 wherein the host cell is a hamster or a mouse-myeloma cell, especially a CHO- or BHK-cell or a NSO cell.

40. Use of a producer host cell line according to claim 37-39 for biopharmaceutical protein manufacturing.
